# EUROPEAN PATENT APPLICATION

(11) **EP 4 388 933 A1**
(43) Date of publication of application: **26.06.2024**
(21) Application number: 22216531.8
(22) Date of filing: 23.12.2022
(51) Int. Cl.: A46B 15/00, A61C 17/22, A61B 5/00

(54) **ORAL HEALTH CARE**

(71) Applicant: Koninklijke Philips N.V., 5656 AG Eindhoven (NL)
(72) Inventor: HORSTMAN, Pieter, Eindhoven (NL); GOTTENBOS, Bart, Eindhoven (NL); VERHAGEN, Rieko, 5656AG Eindhoven (NL)
(74) Representative: Philips Intellectual Property & Standards

(57) **Abstract**

Proposed concepts thus aim to provide schemes, solutions, concept, designs, methods and systems pertaining to assisting an oral health care routine of a user. In particular, embodiments aim to determine at least one operating parameter value of an oral care device based on: (i) the motion and/or position of an oral care device during performance of the oral health care routine; and (ii) measurements of plaque at locations in the oral cavity of the user. From analysis of the movements and/or location of the oral care device and plaque in the oral cavity of the user, insights into how well the user brushed their teeth, i.e. spending the right amount of time on each tooth, may be derived. Operation of the oral care device may then be automatically controlled, helping to ensure adequate or improved cleaning of a region that may be neglected by the user for example.

## Description

### FIELD OF THE INVENTION

This invention relates to the field of oral health care routines, and in particular to the field of assisting an oral health care routine of a user.

### BACKGROUND OF THE INVENTION

Oral care devices, such as electric toothbrushes or mouthpieces, are used on a regular (e.g. daily) basis, and the oral health care routines involving these devices often involve several complex steps. Thus, the user performing the oral health care routine is often unable to judge their own performance of the routine accurately. A particularly problematic example is that of proper brushing of the teeth.

Guidance and/or coaching for dental hygiene exists, but it is typically provided as a professional service e.g., by dental hygienists. Also, such services have a low frequency of the check-up and therefore are lacking in efficacy.

Currently, some approaches to guided tooth brushing are known and these are typically offered using sensors embedded within the toothbrush such as gyroscopes. However, the guidance provided by such approaches is limited in usefulness and full reliance is placed on a user to follow such guidance.

### SUMMARY OF THE INVENTION

The invention is defined by the claims.

According to examples in accordance with an aspect of the invention, there is provided a control system for an oral care device. The control system comprises: an input interface adapted to obtain: routine data describing at least one of motion and position of the oral care device during performance of an oral health care routine by a user of the oral care device; and plaque data describing measurements of plaque at a plurality of locations in the oral cavity of the user; a processor configured to analyze the routine data and the plaque data to determine oral routine properties; and an output interface adapted to generate, based on the oral care properties, a control signal for controlling at least one operating parameter of the oral care device.

Proposed concepts thus aim to provide schemes, solutions, concept, designs, methods and systems pertaining to assisting an oral health care routine of a user.

In particular, embodiments aim to determine at least one operating parameter value of an oral care device based on: (i) the motion and/or position of an oral care device during performance of the oral health care routine; and (ii) measurements of plaque at locations in the oral cavity of the user. From analysis of the movements and/or location of the oral care device and plaque in the oral cavity of the user, insights into how well the user brushed their teeth, i.e. spending the right amount of time on each tooth, may be derived. Operation of the oral care device may then be automatically controlled, helping to ensure adequate or improved cleaning of a region that may be neglected by the user for example.

In other words, it is proposed that detection movement and/or location of an oral care device during performance of an oral health care routine may be analyzed in conjunction with information about plaque in the user's oral cavity (the may have been previously acquired for example) to obtain information about the user's oral routine (i.e. oral routine properties) that can then be leveraged to determine at least one operating parameter value of the oral care device. That is, insights into the user's performance of an oral health care routine may be derived based on (prior and/or current) measurements of plaque in the oral cavity of the user and movements/positioning of their oral care device during use. Such measurements of plaque may be obtained using a sensor incorporated into the oral care device. Alternatively, plaque measurements may be obtained using a separate, dedicated device (e.g. before or after performance of the oral routine) and/or retrieved from a database of such measurements.

By providing a concept for analyzing oral health care routines in consideration of a user's plaque and their operation (manipulation) of an oral care device during cleaning, feedback analogous to guided brushing may be provided to users, and the guidance may be automatically implemented through altered operation of the oral care device. Reliance on the user following guidance may thus be avoided by the proposed concept(s).

Embodiments may be employed in conjunction with toothbrushes. However, embodiments are not limited to the oral care device comprising a toothbrush. For example, the oral care device may comprise a mouthpiece, a flossing device, or any other personal oral care device. One or more proposed concept(s) may therefore be employed in a range of different oral care devices. Embodiments may therefore have wide application in the field of oral care devices and be of relevance to dentistry propositions. For example, by enabling automatically improved or optimized operation of an oral care device, embodiments may improve cleaning of a user's teeth, gum, tongue, etc. Such improved cleaning may be based on adapting the operation of a user's oral care device according to the presence and/or variation of plaque throughout the user's oral cavity. Accordingly, embodiments may be used in relation to dental treatment so as to support a dental care professional when providing treatment for a subject.

By being integrated into the normal brushing regiment of a user, embodiments may support improved dental care. Improved oral health care routines may therefore be provided by proposed concepts.

For instance, by automatically analyzing the variation of plaque and user's mouth in combination with the motion and/or location of an oral care device during performance of an oral health care routine, one or more oral routine properties may be determined. From the oral routine properties, operating parameters of the oral care device may be determined and automatically controlled, thus adapting cleaning parameters/performance to the plaque variations in the user's mouth.

An example oral routine property may, for instance, pertain to user bias. For instance, if the oral health care routine is the user brushing their teeth, the oral routine parameter value may be a ratio of time spent brushing the left side of the mouth to time spent brushing the right side of the mouth, or alternatively, percentage of the mouth cleaned. Such insights may enable the adaptation of the oral care device's operation when positioned in the left side of the mouth, such that improved cleaning of the left side of the mouth is achieved.

Ultimately, an improved performance of an oral health care routine by a user may be supported by the proposed concept(s).

Information on the motion and/or position of the oral care device during performance of an oral health care routine by the user may, for example, be obtained from video of the user and/or the oral care device captured during performance of an oral health care routine. That is, in an example of a user brushing their teeth, video may be captured of the user brushing their teeth and then analyzed. Such video may be obtained using existing or conventional devices that include cameras already owned by a user. Such use of video data to derive insights into a user's performance of an oral health care routine may permit assistance to the user in their routine without the need for dedicated sensors in the oral care device, which may be costly and increase the complexity of the required oral care device. Thus, assistance may be provided to users performing oral health care routines regardless of whether or not the oral care device involved has sensors suitable for monitoring an oral health care routine.

In some embodiments, the at least one operating parameter value may comprise at least one of: an operating frequency; a vibration amplitude; a treatment intensity; an operating duration; a pace time value; an operating power; a motion direction; an operating angle; a vibration duration or pattern; a feedback signal value; a user interface status; an indicator value; and a user interface element value. Various aspects of the operation an oral care device may therefore be controlled by proposed embodiments. Also, operation of the oral care device may be controlled for automatically improved/optimized cleaning and/or for providing feedback to the user (e.g. via an audible, visual or tactile/haptic feedback signal). For instance, the at least one parameter value may comprises a feedback signal value defining an audible, visual or haptic feedback signal to be communicated by the oral care device in this way, many different types of oral care devices may be supported and/or a wide range of operating variables of an oral care device may be controlled for improved or optimal cleaning via automated control and/or user feedback.

In an embodiment, the oral routine properties may comprise one or more of: user bias value; a measure of completion of the oral health care routine; a measure of completion of a subroutine of the oral health care routine; and a time duration, and preferably wherein the oral routine properties are location-dependent. An example of user bias may be a ratio of time spent brushing the upper teeth to the time spent brushing the lower teeth. An example of a measure of completion of the oral health care routine may be a number of teeth remaining to be properly cleaned. An example of a measure of completion of a subroutine of the oral health care routine may be whether the user has flossed. An example of time duration may be the total time spent brushing each region of the mouth. These oral routine properties may enable the automatic adaptation/control of one or more operating parameters of the user's oral care device, such that operation of the oral care device during use is adapted to provide improve cleaning without the need for the user to consciously change/adapt their normal cleaning routine. By way of example, a user-perceivable signal may normally be supplied after 30 seconds as an indicator to move to the next quadrant or the oral cavity. Based the determined oral routine properties, a control signal may be generated for altering the timing of the signal, e.g. for a quadrant with more plaque, embodiments may control the signal to be supplied after 40 seconds for that quadrant, thus causing the user to clean the quadrant for a longer duration.

In some embodiments, the processor may be configured to provide the routine data and the plaque data as input to a machine learning algorithm, the machine learning algorithm being trained to predict, for the oral health care routine associated with the routine data, at least one property of the oral health care routine. The machine learning algorithm may, for example, comprise a supervised classifier model. This may be especially useful for classifying detected behavior, such as brushing specific regions of a user's mouth.

Further, in an embodiment, the machine learning algorithm may be trained using a training algorithm configured to receive an array of training inputs and respective known outputs, wherein a training input comprises routine data associated with an oral health care routine and plaque data describing measurements of plaque at a plurality of locations in the oral cavity of the user associated with the user, and wherein a respective known output comprises at least one property of the oral health care routine. This allows the machine learning algorithm to become especially proficient at determining property values of oral health care routines.

The machine learning algorithm may comprise a pretrained model further trained on routine and plaque data that has been manually or automatically annotated.

In some embodiments, the processor may be configured to provide the motion data and plaque data as input to a rule-based algorithm designed to predict, for the oral health care routine associated with the routine data, at least one property of the oral health care routine. These property values to be deduced from the motion data without the use of a machine learning algorithm which may ease computational demands.

In some embodiments, analyzing the routine data and the plaque data may comprise: predicting, based on the motion data, a location of contact between the oral care device and a dental surface of the user; determining, based on the plaque data, a plaque value for the predicted location of contact; and determining, based on the determined plaque value for the predicted location of contact, an oral routine property for predicted location of contact. The output interface may then be adapted to generate, based on the determined plaque value for the predicted location of contact, a control signal for controlling at least one operating parameter of the oral care device. For example, in the case of a user brushing their teeth, from the location of contact between the personal care device and a surface of the user, the region of brushing taking place can be deduced. For instance, it can be deduced the user is currently brushing their left molar. Further, from the plaque value for the region (e.g. left molar) the user's brushing performance for that region may be deduced. Operation of the oral care device may then be automatically adapted when brushing the same region, according to the user's brushing performance for the region.

According to another aspect of the invention, there is provided a computer-implemented method of controlling an oral care device. The method comprises: obtaining routine data describing at least one of motion and position of the oral care device during performance of an oral health care routine by a user of the oral care device; obtaining plaque data describing measurements of plaque at a plurality of locations in the oral cavity of the user; analyzing the routine data and the plaque data to determine oral routine properties; and generating, based on the oral care properties, a control signal for controlling at least one operating parameter of the oral care device.

Analyzing the routine data and the plaque data may comprise: providing the routine data and the plaque data as input to a machine learning algorithm, the machine learning algorithm being trained to predict, for the oral health care routine associated with the routine data, at least one property of the oral health care routine,

In some embodiments, analyzing the routine data and the plaque data may comprise: predicting, based on the motion data, a location of contact between the oral care device and a dental surface of the user; determining, based on the plaque data, a plaque value for the predicted location of contact; and determining, based on the determined plaque value for the predicted location of contact, an oral routine property for predicted location of contact.

In some embodiments, there is provided a computer program comprising code means for implementing any of the methods described above when said program is run on a processing system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for controlling an oral care device according to a proposed embodiment;
Fig. 2 is a more in-depth flow diagram of a method for controlling an oral care device according to a proposed embodiment;
Fig. 3 depicts a simplified block-diagram of the key information flow, inputs and outputs of an exemplary embodiment;
Fig. 4 is a simplified block diagram of a system for controlling an oral care device according to a proposed embodiment; and
Fig. 5 illustrates an example of a computer within which one or more parts of an embodiment may be employed

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figs are merely schematic and are not drawn to scale. It should also be understood that the same reference numerals are used throughout the Figs to indicate the same or similar parts.

Implementations in accordance with the present disclosure relate to various techniques, methods, schemes and/or solutions pertaining to assisting an oral health care routine of a user. According to proposed concepts, a number of possible solutions may be implemented separately or jointly. That is, although these possible solutions may be described below separately, two or more of these possible solutions may be implemented in one combination or another.

Embodiments of the invention aim to assist an oral health care routine of a user by analyzing: (i) measurements of plaque at a plurality of locations in the oral cavity of the user; and (ii) motion (i.e. movement) and/or location of an oral care device during performance of the oral health care routine by the user. The analysis determines oral routine properties that can then be used to control at least one operating parameter of the oral care device.

Proposed concepts thus aim to determine at least one operating parameter of an oral care device based on plaque information and motion of an oral care device during performance of an oral health care routine.

Information on the motion and/or location of the oral care device may, for example, be obtained from video of the user and/or the oral care device captured during performance of an oral health care routine. That is, in an example of a user brushing their teeth with a toothbrush, video may be captured of the user brushing their teeth and then analyzed along with information about the user's plaque distribution within their oral cavity. From analysis of the plaque and movements of the toothbrush, insights into how well the user brushes their teeth, i.e. spending the right amount of time on each tooth, may be derived. Operation of the toothbrush may then be automatically controlled, so as to improve or optimize cleaning according to location of the brush head for example.

Thus, there may be proposed concepts for assisting an oral health care routine of a user, and this may be done based on spatially-dependent plaque information and detected motion and/or position of the oral care device of during performance of the oral health care routine. Adapting/controlling operating parameter values of the oral health care routine may help to automatically optimize without the user needing to adjust their normal cleaning routing and/or follow complicated cleaning guidance.

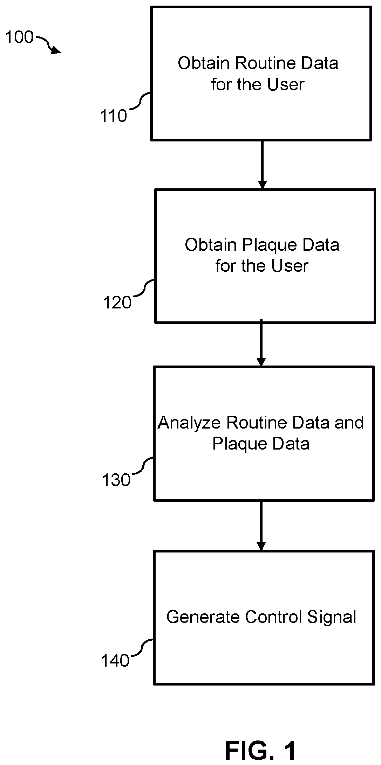

Referring now to Fig. 1, there is depicted a flow diagram of a method 100 for controlling an oral care device according to a proposed embodiment.

The method begins with the step 110 of obtaining routine data describing at least one of motion and position of the oral care device during performance of an oral health care routine by a user of the oral care device. By way of example, routing data may describe: location of contact, contact pressure (from a pressure sensor), the brushing angle (measured from multi axis force sensors), time spent at location; the motion (e.g. scrubbing motion); velocity of the brush, etc. Such data can be obtained using existing or conventional devices that include cameras already owned by a user. For instance, a conventional device may be one of: a smartphone, a tablet, a laptop, or any other suitable device. For the example of a computer vision-based method of analyzing oral health care routines, proposed embodiments may be provided to users irrespective of the type of the toothbrush or personal care device being used. The use of video data alone to derive insights into a user's performance of an oral health care routine may permit automatic assistance to the user in their routine without the need for dedicated sensors in the oral care device (which are costly and increase the complexity of the required oral care device). Thus, assistance may be provided to users performing oral health care routines regardless of whether or not the oral care device involved has sensors suitable for monitoring motion and position of an oral care device during performance of an oral health care routine.

In step 120, plaque data describing measurements of plaque at a plurality of locations in the oral cavity of the user is obtained. Such data can be obtained using existing or conventional devices that include plaques sensors for measuring plaque at differing locations within an oral cavity. For instance, embodiments of the invention may leverage plaque detection technology such as a quantitative light-induced fluorescence (QLF) technique, a Fluorescence Imaging with Reflectance Enhancement (FIRE) technique, white light polarization sensitive imaging, laser scanning microscopy, structured light imaging, or alternative techniques. Furthermore, the embodiments may employ a localization technique or a combination of localization techniques that enable to register the obtained plaque sensor information in the mouth of the user. Localization techniques may comprise inertial sensors such as IMUs, GPSs, Gyroscopes, magnetic field sensors such as Hall sensors, GMR sensors etc., but may also include digital image stitching techniques such as SLAM (simultaneous localization and mapping) or SIFT (Scale Invariant Feature Transform) that connect adjacent images formed by the plaque sensing technology to form a consistent map of the features identified in the user's oral cavity (i.e. mouth).

In step 130, the obtained routine data and plaque is analyzed to determine at least one oral routine property. An example property may, for instance, pertain to user bias. For instance, if the oral health care routine is the user brushing their teeth, the oral routine property may be a ratio of time spent brushing the left side of the mouth to time spent brushing the right side of the mouth, or alternatively, percentage of the mouth cleaned. Such insights may enable the adaptation an operating parameter of the oral cleaning device, to compensate for oral routine property and thus improve cleaning efficacy.

In some embodiments, the at least one oral routine property may comprise at least one of: user bias value; a measure of completion of the oral health care routine; a measure of completion of a subroutine of the oral health care routine; and a time duration. Such oral routine properties may also be location-dependent. An example of user bias may be a ratio of time spent brushing the upper teeth to the time spent brushing the lower teeth. An example of a measure of completion of the oral health care routine may indicate teeth remaining to be properly cleaned. An example of a measure of completion of a subroutine of the oral health care routine may be whether the user has flossed. An example of time duration may be the total time spent brushing each region of the mouth.

In step 140, a control signal for controlling at least one operating parameter of the oral care device is generated based on the at least one oral care property determined in step 130. The at least one operating parameter value may comprise at least one of: an operating frequency; a vibration amplitude; a treatment intensity; an operating duration; a pace time value; an operating power; a motion direction; an operating angle. In this way, one or more operating parameters of the oral care device can be controlled to take account of the one or more oral routine properties and thus optimize cleaning, e.g. compensate for deficiencies in a user's performance of the oral health care routine, without user involvement or guidance.

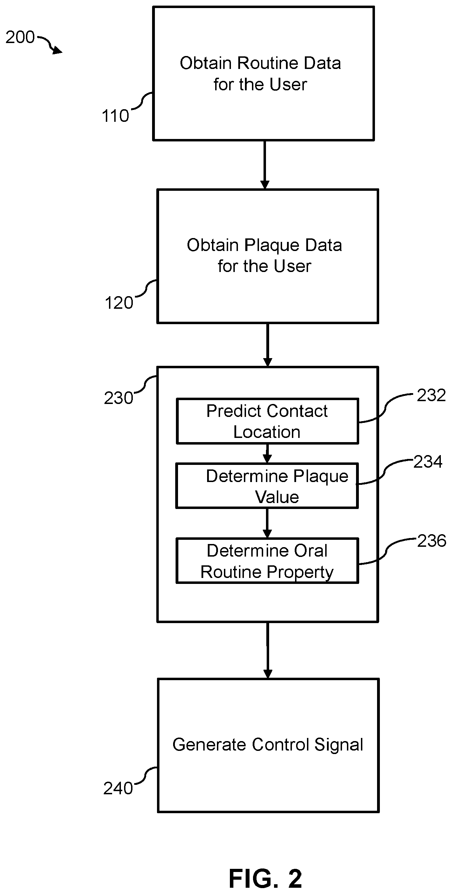

Referring now to Fig. 2, there is depicted a more in-depth flow diagram of a method 200 for assisting an oral health care routine of a user according to an exemplary embodiment. The method begins with the steps 110 and 120 of obtaining routine data and plaque data, as described above.

In step 230, the obtained routine data and plaque data is analyzed to determine at least one oral routine property. Here, step 230 of analysis comprises the sub-steps: (step 232) predicting, based on the motion data, a location of contact between the oral care device and a dental surface of the user; (step 234) determining, based on the plaque data, a plaque value for the predicted location of contact; and (step 236) determining, based on the determined plaque value for the predicted location of contact, an oral routine property for predicted location of contact. Embodiments may thus analyze measured plaque maps and provide meaningful and personalized guidance to the user based on the location, historical trends in the measurement and brushing data, and metadata that can be derived from both internal as well as external sources (e.g., the user's dentist,) such as e.g., age, location, gender, previous oral health issues, and systemic aspects that are relevant for oral hygiene such as e.g., pregnancy, diabetes, dry mouth, etc.

Then, in step 240, the output interface generates, based on the determined plaque value for the predicted location of contact, a control signal for controlling at least one operating parameter of the oral care device. For example, in the case of a user brushing their teeth, from the location of contact between the personal care device and a surface of the user, the region of brushing taking place can be deduced. Further, from the plaque value for the region, the user's brushing performance for that region may be deduced. The control signal may then comprise information for controlling an operating parameter of the oral care device when brushing the same region (e.g. according to the user's brushing performance for the region).

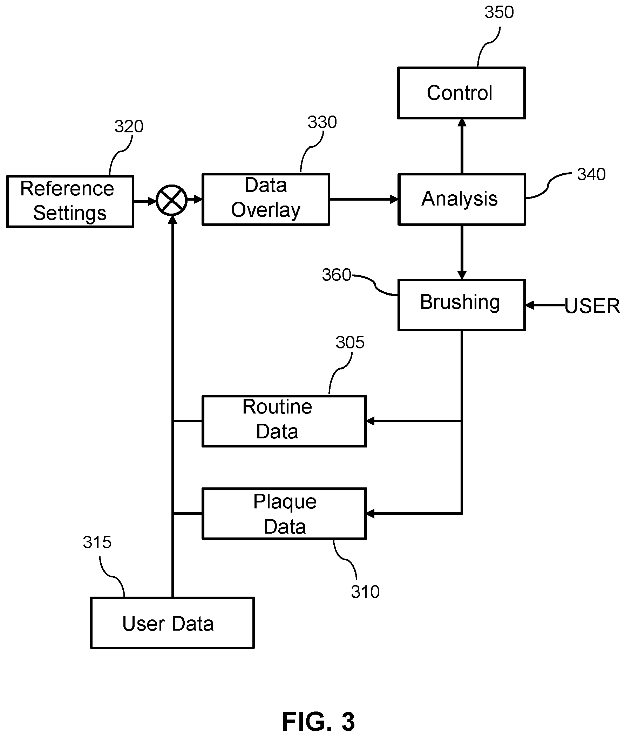

By way of further example of the proposed concept(s), Fig. 3 depicts a simplified block-diagram of the key information flow, inputs and outputs of an exemplary embodiment.

Routine data 305, plaque data 310 and user data 315 is combined with reference setting 320 by a data overlay process 330. The combined data is analyzed in analysis process 340 and results in the provision of a control signal 350 for controlling one or more operating parameters for an electric powered toothbrush.

The user of the toothbrush undertakes a brushing routine 360 which results in the provision of updated routine data and plaque data that is then provided in a feedback loop for updated analysis and control signal generation.

The following operating parameters of an oral care device may be automatically controlled (i.e. modified, adjusted, etc.) by a control signal generated according to the proposed concept(s):
- pace-timer (e.g. user feedback timer signal for pacing brushing) to vary the amount of time spent on a certain area
- audible/visible feedback signal to guide user on brushing parameter such as applied pressure, brushing direction, brushing angle, brushing motion or pattern, movement speed, etc.
- motor power to increase the friction in a certain area
- amplitude of vibration
- motion direction, e.g., a linear to rotational
- angle of the brush head relative to handle
- device functionality operation, e.g. air-floss, oral-irrigation, etc.

An existing electric toothbrush (the Sonicare (TM) 9000 series toothbrush) comprises an integrated IMU for detecting the brushing location in the mouth during use (i.e. routine data). Embodiments may leverage this data along with plaque data from an additional attachment containing a plaque sensor (which may be used on a regular basis for making images of the teeth using one of the abovementioned plaque detection modalities). Alternatively, a plaque sensor may be integrated in the power toothbrush. Images can be temporarily stored either on the attachment, on the PTB handle or on the PTB base station/docking charger to be interpreted locally or upload to the cloud for processing. In this way, IMU data combined with image feature analysis can be used to reconstruct complete plaque maps of a user's oral cavity. Based on such maps, information can be extracted on how, and with what modality, plaque deposits can be effectively removed. Using this information, one or more operating parameters of the oral care device may be controlled. Exemplary operating parameters include user interface operation parameters that may be controlled to coach the user to remove the detected plaque better: e.g. sounds, voice feedback, lights, displays, haptic feedback on the handle (hand) or the brush head (mouth, e.g. a buzz).

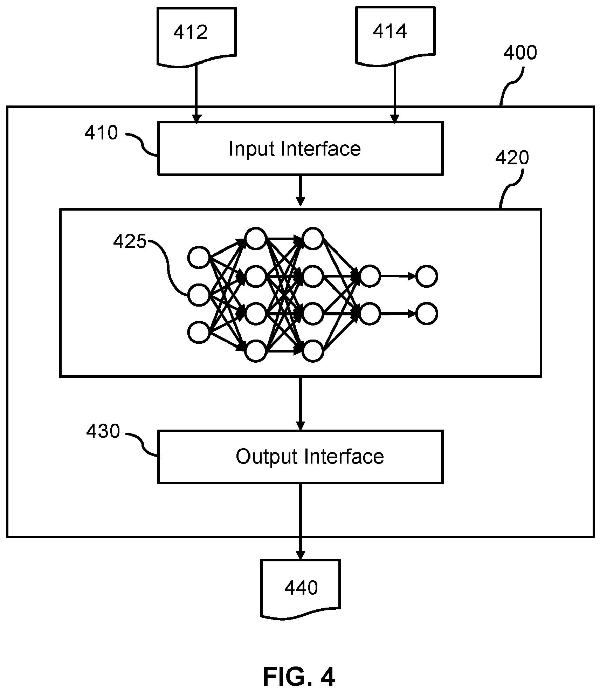

Referring now to Fig. 4, there is depicted a simplified block diagram of a control system 400 for an oral care device according to an embodiment. The control system 400 comprises an input interface 410, one or more processors 420 and an output interface 430.

The input interface 410 receives: routine data 412 describing at least one of motion and position of the oral care device during performance of an oral health care routine by a user of the oral care device; and plaque data 414 describing measurements of plaque at a plurality of locations in the oral cavity of the user.

The one or more processors 420 analyze the routine data and the plaque data to determine one or more oral routine properties. Specifically, the processor is configured to provide the routine data and the plaque data as input to a machine learning algorithm (e.g. neural network) 425. The machine learning algorithm 425 is trained to predict, for the oral health care routine associated with the routine data, at least one property of the oral health care routine.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). This exemplary embodiment of the present invention employs CNN-based learning algorithms. CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries for the machine learning algorithm 425 in method correspond to example routine data and plaque data. The training output data entries correspond to one or more properties of the oral health care routine. Further, several pre-processing methods may be employed to improve the training samples. In other words, the fi machine learning algorithm 425 can be trained using a training algorithm configured to receive an array of training inputs and respective known outputs, wherein a training input comprises routine data associated with an oral health care routine and plaque data describing measurements of plaque at a plurality of locations in the oral cavity of the user associated with the user, and wherein a respective known output comprises at least one at least one property of the oral health care routine.

In some embodiments, the machine learning algorithm 425 is a pretrained model further trained data that has been manually annotated. This allows the machine learning algorithm 425 to become especially proficient at identifying oral care properties. In some embodiments, the pretrained model is trained on a COCO dataset based on RESNET34, RESNET 50, and/or RESNET101 backbone architecture.

In some embodiments, the first CNN is more specifically a Mask-RCNN deep neural based model trained on custom annotations.

Based on the oral care properties determined by the processor(s) 420, the output interface 430 a control signal for controlling at least one operating parameter of the oral care device.

By way of yet further example, a control system according to a proposed embodiment (such as the example of Fig. 4 described above) may be incorporated into an oral care device, such as a toothbrush or mouthpiece. That is, embodiments of the invention may provide an oral care device comprising a control system according to one or more of the proposed concepts.

Alternatively, a control system according to a proposed embodiment (such as the example of Fig. 4 described above) may be provided as a separate, standalone device/system that can be employed for controlling one or more oral care device. Thus, embodiments of the invention may provide an oral care system comprising: an oral care device; and a control system according to a proposed embodiment for controlling at least one operating parameter of the oral care device.

Also, a control system according to a proposed embodiment (such as the example of Fig. 4 described above) may further comprise a plaque sensor configured to detect plaque at a plurality of locations in an oral cavity of a subject. In this way, the control system may acquire plaque data by taking measurements of plaque at various locations within a user's oral cavity. Other embodiments may, however, obtain plaque data from a separate source of plaque data (e.g. database or data repository) that contains pre-acquired plaque data describing measurements of plaque at a plurality of locations in the oral cavity of the user.

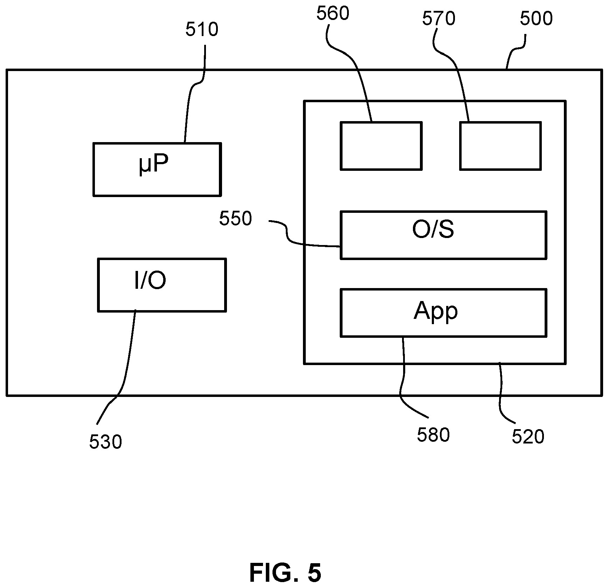

Fig. 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor-based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 800 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figs illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figs. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figs. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A control system (400) for an oral care device, the control system comprising:
an input interface adapted to obtain:
routine data (412) describing at least one of motion and position of the oral care device during performance of an oral health care routine by a user of the oral care device; and
plaque data (414) describing measurements of plaque at a plurality of locations in the oral cavity of the user;
a processor (420) configured to analyze the routine data and the plaque data to determine one or more oral routine properties; and
an output interface (430) adapted to generate, based on the oral care properties, a control signal (440) for controlling at least one operating parameter of the oral care device.

2. The control system of claim 1, wherein the at least one parameter value comprises at least one of: an operating frequency; a vibration amplitude; a treatment intensity; an operating duration; a pace time value; an operating power; a motion direction; an operating angle; a vibration duration or pattern; a feedback signal value; a user interface status; an indicator value; and a user interface element value.

3. The control system of claim 2, wherein the at least one parameter value comprises a feedback signal value defining an audible, visual or haptic feedback signal to be communicated by the oral care device.

4. The control system of any of claims 1 to 3, wherein the plaque data (414) comprises historical plaque data describing measurements of plaque at a plurality of locations in the oral cavity of the user obtained before the performance of the oral health care routine by the user.

5. The control system of any of claims 1 to 4, wherein the oral routine properties comprise one or more of: user bias value; a measure of completion of the oral health care routine; a measure of completion of a subroutine of the oral health care routine; and a time duration, and preferably wherein the oral routine properties are location-dependent.

6. The control system of any of claims 1 to 5, wherein the processor (420) is configured to:
provide the routine data and the plaque data as input to a machine learning algorithm(425), the machine learning algorithm being trained to predict, for the oral health care routine associated with the routine data, at least one property of the oral health care routine,
and optionally wherein the machine learning algorithm comprises a supervised classifier model.

7. The control system of claim 5, wherein the machine learning algorithm (425) is trained using a training algorithm configured to receive an array of training inputs and respective known outputs, wherein a training input comprises routine data associated with an oral health care routine and plaque data describing measurements of plaque at a plurality of locations in the oral cavity of the user associated with the user, and wherein a respective known output comprises at least one at least one property of the oral health care routine.

8. The control system of any of claims 1 to 5, wherein the processor (420) is configured to provide the motion data and plaque data as input to a rule-based algorithm designed to predict, for the oral health care routine associated with the routine data, at least one property of the oral health care routine.

9. The control system of any of claims 1 to 8, wherein the processor (420) is configured to:
predict, based on the motion data, a location of contact between the oral care device and a dental surface of the user;
determine, based on the plaque data, a plaque value for the predicted location of contact; and
determine, based on the determined plaque value for the predicted location of contact, an oral routine property for predicted location of contact,
and wherein the output interface is adapted to generate, based on the determined plaque value for the predicted location of contact, a control signal for controlling at least one operating parameter of the oral care device.

10. An oral care device comprising a control system according to any of claims 1 to 9.

11. An oral care system comprising:
an oral care device; and
a control system according to any of claims 1 to 9 for controlling at least one operating parameter of the oral care device.

12. The oral care system of claim 11, further comprising:
a plaque sensor configured to detect plaque at a plurality of locations in an oral cavity of a subject.

13. A computer-implemented method for controlling an oral care device, comprising:
obtaining (110) routine data describing at least one of motion and position of the oral care device during performance of an oral health care routine by a user of the oral care device;
obtaining (120) plaque data describing measurements of plaque at a plurality of locations in the oral cavity of the user;
analyzing (130) the routine data and the plaque data to determine one or more oral routine properties; and
generating (140), based on the oral care properties, a control signal for controlling at least one operating parameter of the oral care device.

14. The method of claim 13, wherein analyzing the routine data and the plaque data comprises:
providing the routine data and the plaque data as input to a machine learning algorithm, the machine learning algorithm being trained to predict, for the oral health care routine associated with the routine data, at least one property of the oral health care routine,

15. A computer program comprising code means for implementing the method of any preceding claim when said program is run on a processing system.
